# EUROPEAN PATENT APPLICATION

(11) **EP 4 030 104 A1**
(43) Date of publication of application: **20.07.2022**
(21) Application number: 21151658.8
(22) Date of filing: 14.01.2021
(51) Int. Cl.: F24C 15/20

(54) **EXTRACTION DEVICE**

(71) Applicant: ELECTROLUX APPLIANCES AKTIEBOLAG, 105 45 Stockholm (SE)
(72) Inventor: BENOLD, Frank, 33080 Porcia PN (IT); SCHNEIDER, Nikolai, 91541 Rothenburg (DE)
(74) Representative: Electrolux Group Patents

(57) **Abstract**

The present invention concerns an extraction device (14) for removing air from an area above a cooking hob (12), which comprises an air inlet opening (24), a fan (36) for aspirating air from the area above the cooking hob (12) through the air inlet opening (24), a filter (60) element and an outlet opening (30) for exhausting air. According to the invention, the filter element (60) is removably arranged in a filter seat (62) downstream of the air inlet opening (24) and is accessible through the air inlet opening (24). Support means or actuation means are included supporting or facilitating insertion into and/or removal from the filter seat (62). The support means or actuation means are arranged at and/or act on the filter element (60) in a direction at least partially opposite to the direction of insertion.

## Description

The present invention relates to an extraction device for removing air, specifically cooking vapours, from an area above a cooking hob according to the preamble of claim 1.

During the performance of cooking activities under use of a cooking hob, cooking vapours are generated, which are distributed over the cooking area. In order to avoid these cooking vapours to be spread throughout the entire kitchen space, an arrangement of an extraction device, particularly an extractor hood, is common. Said extraction device is operated in parallel to the cooking hob, thereby sucking in those cooking vapours in order to filter out particles and tiny droplets from the vapours and/or to blow the vapours to the outside of the building. While it has been common to arrange such an extractor hood above the cooking area, in recent years downdraft extraction devices have been finding a growing market. Such kind of extraction device is usually arranged below a worktop of the related cooking hob and it comprises at least one fan for sucking air from the cooking area through an opening or recess arranged in the worktop.

It is quite common for domestic extraction devices to operate with recirculating air, which means that the aspirated air is not extracted outside of the installation room or building, but is blown out into a kitchen cabinet or directly into the ambient air after it has been filtered. A usual order of arrangement of devices is as follows: the air with cooking vapours is aspirated from the cooking zones above the worktop of the cooking hob, subsequently passing a particle filter, e. g. a grease particle filter. Then, after having passed the fan, the conveyed air passes an odour filter before being blown out through an outlet opening. Particularly in the case of downdraft extraction devices, servicing activities on the odour filter are cumbersome due to the fact that this filter is arranged close to the outlet opening, which is usually situated on a level of or arranged in a plinth or the kitchen cabinet.

There are also extraction devices known on the market, which comprise an odour filter that is accessible through an inlet opening for an entry of air including cooking vapours. However, the respective odour filter is usually arranged with a lateral displacement from an engagement space below the inlet opening and a gripping of the filter element for pulling it out of its filter seat is cumbersome.

It is an object of the present invention to provide an extraction device, which is designed in a way allowing a simplified access to a filter element, specifically to an odour filter, and its facilitated removal from a filter seat, e. g. for servicing activities.

The object is achieved for an extraction device according to the preamble of claim 1 by the features of the characterizing part of claim 1.

An extraction device for removing air from an area above a cooking hob comprises an inlet opening for an entry of the air, a fan for aspirating the air from the area above the cooking hob through the air inlet opening, a filter element for a treatment of the aspirated air and an outlet opening for exhausting air, in particular into the ambient air. The air inlet opening is particularly arranged at or below a cutout or a recess of a worktop, which specifically may be a cooktop or glass plate of the cooking hob. The aspiration of the air from the area above the cooking hob is particularly performed through the cutout or recess of the worktop. The filter element may comprise an odour filter, specifically a carbon filter, for removing odours from the aspirated air, and/or a grease particle filter for removing particles from the aspirated air, and/or an electrostatic filter for removing particles, which are based on the electrostatic principle, from the aspirated air. According to the present invention, the filter element is removably arranged in a filter seat downstream of the inlet opening and is accessible through the air inlet opening. A preferred location for the arrangement is formed by the interior of the extraction device. Particularly, access to the filter element is provided through the cutout or recess of the worktop. More specifically, a receiving space may be arranged directly downstream of the inlet opening and the access to the filter element is provided through this receiving space, specifically after passing through the inlet opening. Support means or actuation means are included in the extraction device, which support or facilitate the insertion of the filter element into and/or the removal of the filter element from the filter seat. In particular, a manual insertion and/or removal is supported or facilitated by the support or actuation means. Said support means or actuation means are arranged at and/or act on the filter element in a direction at least partially opposite to the direction of insertion. Preferably, the support means and/or actuation means are arranged at an end or side of the filter element, which end or side faces the direction of the insertion movement or of the removal movement and/or act on an end or side of the filter element in a direction at least partially opposite to the direction of insertion. Face the direction may be understood in that the respective side or end is perpendicularly penetrated by an axis of the filter element, which axis is in parallel arrangement with the direction of insertion or removal movement.

The filter element may be loosely arrangeable in the filter seat. For a safe and defined positioning, however, the filter element is lockable in the filter seat. To this end, the filter seat and/or the filter element preferably comprise(s) a locking mechanism, which is activated in an operating position of the filter element that it assumes when it is inserted in the filter seat at its defined position in which it is fully effective. This operating position, or locked position, may be received by a user's manual actuation onto the filter element and particularly against a force exerted by the support or actuation means during the insertion movement. One specific embodiment may also provide for a filter element and/or support means or actuation means having a locking mechanism, which comprise snapping means, clicking means and/or latching means. According to a particularly preferred embodiment, the support means or actuation means are connected to or integrated in the locking mechanism.

In particular, the extraction device is an extraction hood, which may be installed in a household kitchen, but an arrangement in an industrial or canteen kitchen may be possible as well. The sucked or aspirated air, which is or shall be removed from the cooking hob, specifically include cooking vapours, which notably arise during cooking with uncovered cookware. Moreover, the extraction device is particularly a downdraft extraction hood and/or it may form a combination appliance in combination with the cooking hob. More specifically, the extraction device in such a combination appliance is arranged below the cooking hob.

The present invention is favourable in view of many respective appliances, notably combination appliances, on the market. A particularly simplified access to the filter element, particularly the odour filter, is provided by a structure or design of the extraction device, which allows the filter element to be removed through the air inlet opening or recess. That way, the user of the combination appliance only needs to gain access to the interior of the appliance through the recess or air inlet opening, preferably without any excessive disassembly of any housing parts. Moreover, the provision of support means or actuation means further facilitates access to the filter element in that the handling during servicing activities is simplified.

Another advantage achieved by the present invention is founded in the fact of having a tool-free assembly and/or disassembly of the filter element, specifically of the odour filter. This increases the ease of use during the operation of the extraction device and promotes the user's preparedness for regular maintenance activities, particularly for cleaning or replacing filter elements or filtering means.

The cooking hob being the source for the aspirated cooking vapours may be of any type including electric or gas cooking hobs. In particular, the electric cooking hob is an induction cooking hob. The cooking hob may further be an autarkic cooking appliance, particularly integrated in a countertop of kitchen furniture, or it may be part of a stove.

A top surface of the cooktop or glass plate, which is accessible to a user, may be used for putting cookware thereon, specifically in order to perform a cooking process. To this end, the cooking hob preferably comprises at least one heating element defining a cooking zone on the cooktop or glass plate, which is favourably made out of glass ceramics.

A preferred embodiment of the present invention provides for a filter element, which is arranged downstream of the fan and removable through the air inlet opening, in particular through the cutout or recess of the worktop.

Preferably, the support means or actuation means for facilitating insertion and/or removal of the filter element comprise a guide mechanism, which may be a sliding-block guide, and/or a spring element configured to at least partially displace the filter element from its operating position that it assumes when it is inserted in the filter seat. At least one of the insertion, the removal and the displacement particularly takes place upon a user's manual operation. Said guide mechanism preferably includes a cardioid guide mechanism.

The filter element may be both positionable in and at least partially removable from its operating position within the filter seat, more specifically the operating position that is assumed by the filter element when it is inserted in the filter seat, by an actuation in the direction of insertion. This action is specifically a mechanical action, which may be performed by the user.

According to an embodiment, the support means or actuation means comprise an actuation surface, which is configured for performing a manual insertion and/or removal movement of the filter element, wherein the manual movement is specifically performed by the user. Said actuation surface is particularly a gripping surface, which may be arranged at or on a handle.

A lid or cover may be provided, which separates the filter seat from the inlet opening. More specifically, the spatial separation of the filter seat may be implemented towards a receiving space, which is arranged downstream, particularly directly downstream, of the air inlet opening.

The extraction device according to the present invention may be characterized in that the separation of the filter seat from the inlet opening, or more specifically from the receiving space, is performable in an airtight manner. In this case, the cover comprises in particular a sealing means, which is preferably configured for a radial sealing. Said sealing means specifically comprises an O-ring seal. That way, sealing can be provided independently from an exact position along a displacement path, particularly when a sealing surface is provided along a channel or tube with a constant cross-section.

According to embodiments, a transition section is arranged adjacent to and directly upstream of the filter seat, which transition section is passed through by the filter element during its insertion into the filter seat. In particular, an inner surface of the transition section forms a contact surface for the sealing means. Said transition section favourably extends along the above-mentioned displacement path.

The cover is advantageously fixed to and/or integrally formed with the filter element. In particular, it is fixed to and/or integrally formed with a frame part of the filter element. A preferred solution is characterized in that the cover forms an end portion or end element of the filter element viewed in the direction of insertion movement. In other words, during the insertion of the filter element, the cover forms a final portion or element of the filter element, following the other portions or elements of the filter element in moving direction.

The filter element may be bendable in insertion direction for an adaptation to a curved insertion path. Additionally, or alternatively, the filter element may be incompressible or only marginally compressible in the direction of its movement. On the other hand, non-extensibility of the filter element in the direction of its movement may be provided as well.

The filter element may be included in or supported by a filter carrier or frame. The filter element may be arranged vertically or horizontally in the filter carrier. One specific embodiment is characterized by a filter carrier or filter frame made of a flexible but incompressible material.

An auxiliary means for finding the position of the filter element may be provided to the user, which takes account of the fact that servicing activities are rather rarely to be performed and the user may not always remember said position or the access to the filter element. To this end, the extraction device may comprise a marking or indicator means for pointing out the access to or the position of the filter element to the user. Such kind of marking or indicator means is visible or noticeable through the inlet opening. Moreover, the marking or indicator means particularly provides a visual and/or haptic guidance to the user and/or is preferably located on a bottom cover or an intermediate bottom wall of the extraction device. The user, when looking through the inlet opening, notably faces this marking or indicator means. The marking or indicator means particularly comprise an illumination element, which preferably comprises an LED. More specifically, the illumination element is configured to automatically switch on when the user gains access to the interior of the extraction device through the inlet opening, in particular when a lid or a grid covering the inlet opening is removed from the inlet opening, particularly from the cooktop or glass plate.

One specific embodiment of the present invention is characterized by an insertion aid for the facilitated insertion, notably for a further facilitated insertion, of the filter element into the filter seat. Said insertion aid particularly comprises a rail or track guide means and/or at least one guiding surface. Preferably at least two guiding surfaces and more preferably three guiding surfaces are provided. With the help of this insertion aid a guided insertion is enabled in order to make a smooth insertion possible. By means of the insertion aid, a tilting or an interlocking of the filter element during the insertion movement may be avoided.

More specifically, the insertion aid may be an integral element of the extraction device, so that it may be permanently arranged in the extraction device. Alternatively, it may be a unit or component, which is only used during the servicing process, so that it may be insertable into the extraction device prior to the filter element insertion process, and it may be removable from the extraction device after finalization of the filter element insertion process. The positioning of the insertion aid is particularly executable through the inlet opening.

The filter element may be insertable into and/or removable from the filter seat in an at least approximately horizontal direction and the horizontal extension of the filter element in its direction of movement may be equal to or greater than its vertical extension. A preferred design of the filter element is characterized by a rectangular or oval vertical cross-section of the filter element, wherein said vertical cross-section is defined by the plane or surface resulting from a cut through the filter element perpendicular to its movement direction.

In order to further facilitate servicing activities on the filter element, specifically by enabling extended respective service intervals, purification means for a cleaning of the aspirated air are preferably included. Said purification means may be formed by silver ions that are included in the filter element performing a sanitizing, more specifically an antibacterial and antiviral, effect on the surface or fabric of the filter element. Additionally, or alternatively, in order to receive a similar or an additional effect, lighting means emitting ultraviolet rays may be included in the extraction device, which lighting means irradiate the filter means at least during the operating periods or during at least one time range of an operating period of the extraction device.

Novel and inventive features of the present invention are set forth in the appended claims.

The present invention will be described in further detail with reference to the drawings, in which
- Fig. 1: is a perspective view of a combination appliance comprising a cooking hob and a downdraft extraction device installed in a kitchen cabinet according to a first embodiment;
- Fig. 2: is a cross-sectional perspective view of the disassembled combination appliance of Fig. 1 with a frontal surface cut away;
- Fig. 3: is a rear view of the combination appliance of Fig. 2;
- Fig. 4: is a perspective view of a combination appliance similar to Figs. 1 to 3 from a rear left side according to a second embodiment, illustrating a positioning of a filter element;
- Figs. 5a,b,c: are front, side and top views of the filter element of the combination appliance of Fig. 4;
- Figs. 6a,b,c: are schematic top views of the combination appliance of Figs. 1 to 3 or of Fig. 4 illustrating three stages during an insertion movement of the filter element of Figs. 5a,b,c;
- Fig. 7: illustrates an early stage of a removal movement of the filter element of Figs. 5a,b,c being an opposite movement to the insertion movement illustrated by Figs. 6a,b,c;
- Fig. 8: is a schematic rear view of the kitchen cabinet following Fig. 1, but with rearward exhaust air outlet as illustrated by Fig. 4; and
- Fig. 9: is a rear view of a combination appliance that is modified over the combination appliance according to Fig. 3, forming a third embodiment.

In all figures the same or equivalent part are marked with the same reference numbers.

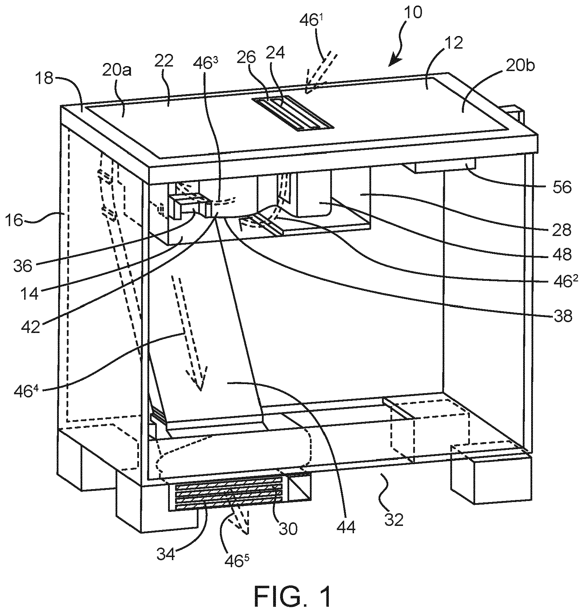

Fig. 1 illustrates a combination appliance 10 comprising a cooking hob 12 and a downdraft extraction device 14 installed in a kitchen cabinet 16. The cooking hob 12 is implemented in a cutout of a kitchen countertop 18 forming a top cover plate of the kitchen cabinet 16. The downdraft extraction device 14 is configured to take away cooking vapours occurring during cooking processes, in particular when cooking with uncovered cookware. The cooking hob 12 comprises cooking regions 20a, 20b arranged on a left half and a right half of a cooktop 22 of the cooking hob 12, which left and right halves are separated from each other by a suction opening 24 for an intake of the cooking vapours, the suction opening 24 being arranged alongside a cooktop centreline. The suction opening 24 is covered by a cover grid 26 for preventing items, e. g. cookware, to fall into the suction opening 24. Instead of the cover grid 26 a lid may be used for entirely covering the suction opening when the extraction device 14 is out of use, but for the operation of the extraction device 14 the lid is pivotable into an upright open position.

A casing 28 of the extraction device 14 is shown in Fig. 1 in transparent illustration. Said casing 28 provides a closed outer shell or first channel for a flow of the sucked-in cooking vapours on their way from the suction opening 24 to an exhaust opening 30 in a base area 32 of the kitchen cabinet 16. Said exhaust opening 30 is also covered, namely by an outlet grille 34.

The flow of the sucked-in cooking vapours through the extraction device 14 is driven by the operation of an extraction fan 36 arranged inside of the casing 28. Said extraction fan 36 comprises a bottom-sided intake opening 38 for sucking the cooking vapours from the interior space of the casing 28.

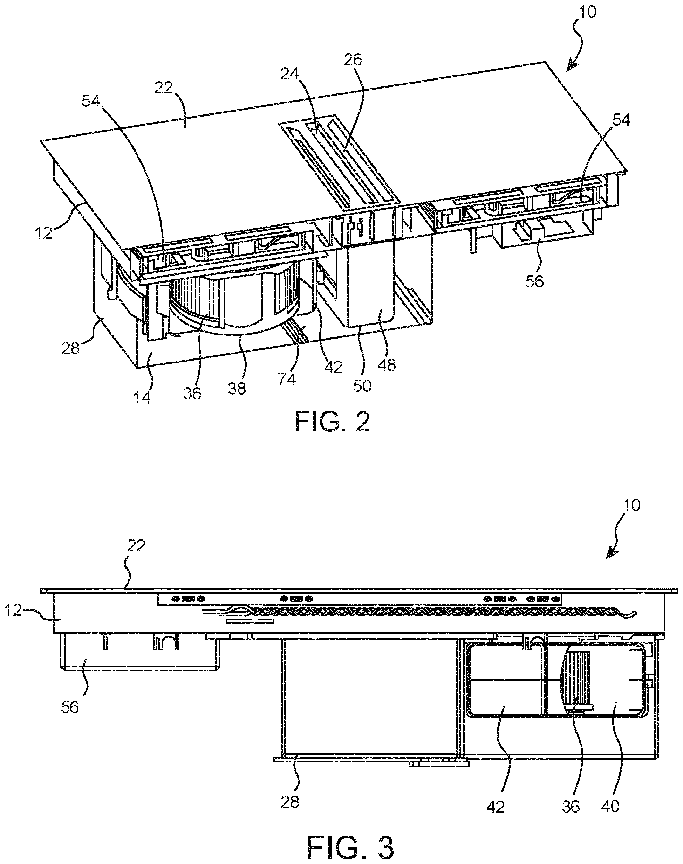

As illustrated by Fig. 3, which is a rear view of the combination appliance 10, a rear-sided fan outlet 40 is arranged for a horizontal exit of the air blown out backwards from the extraction fan housing 42. The fan outlet 40 is connected to a first end of an air duct 44 designed as a rectangular tube and forms a second channel arranged downstream of the above-mentioned first channel. Directly at the passage from the fan outlet 40 to the air duct 44, an air duct bending by 90 degrees is implemented, which redirects the air flow from horizontal to vertical downwards. The air duct 44 may be guided alongside a rear side of the kitchen cabinet 16 and may be bent again by 90 degrees close to a rear lower edge of the kitchen cabinet 16 in order to direct the airflow towards exhaust opening 30 in the base area 32 of the kitchen cabinet 16. Accordingly, the second end of the air duct 44 is connected to the exhaust opening 30. The embodiment illustrated in Fig. 1 shows a solution of the air duct 44 with an inclined section of its downwardly directed portion, directed slightly to the right. Naturally, a solution with said portion arranged in an exact vertical direction is considerable as well.

The course of the cooking vapours from the cooking area through the extraction device 14 to a re-entry into ambient air is illustrated in Fig. 1 by dotted arrows 46¹ to 46⁵. On their way through the extraction device 14, the cooking vapours pass through a filter assembly 48, which is arranged downstream directly behind the suction opening 24 for providing a purification of the conveyed air. Said filter assembly 48 includes a filter carrier 50 supporting a filter element (not shown) that is usually configured for filtering out grease particles and droplets.

The cross-sectional view of Fig. 2 further shows two power boards 54, one for the left cooking region 20a and one for the right cooking region 20b, the power boards 54 providing cooking zones in the left and right cooking regions 20a, 20b with electrical power. In the present embodiment, the cooking hob 12 is an induction cooking hob and the cooking zones are defined by induction coils (not shown) that are arranged below the cooktop 22 of the cooking hob 12. Attached to the bottom side of the power board 54 assigned to the right cooking region 20b, a further circuit board is arranged forming a control electronics 56 for the combination appliance 10.

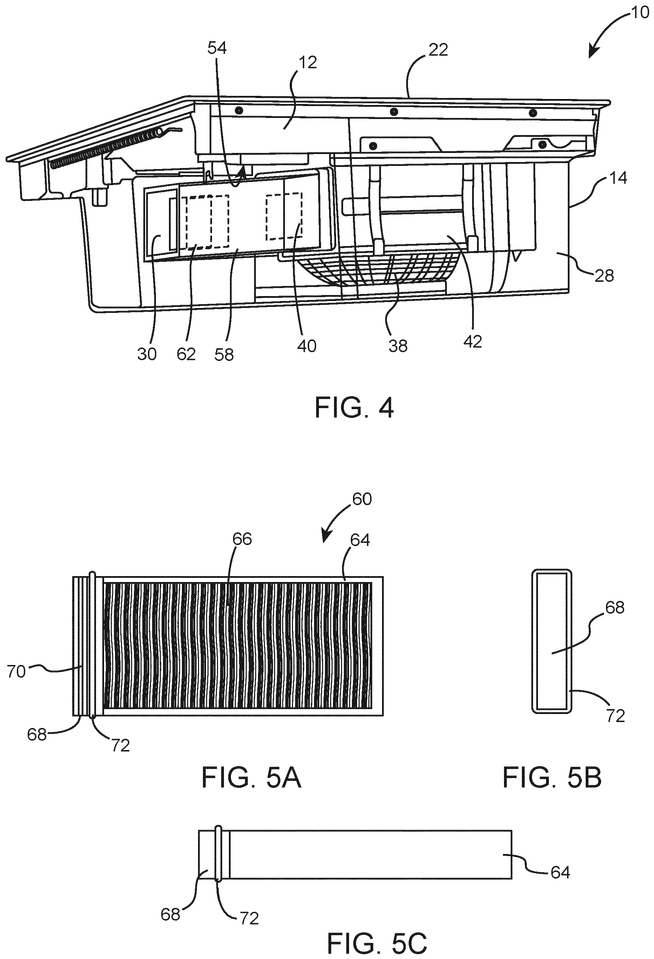

In addition to the afore-mentioned grease filter element included in of the filter assembly 48, the downdraft extraction device 14 comprises a further filter element 60, which is configured for removing cooking odours from the conveyed air. This odour filter element 60 is arranged directly downstream of the fan outlet 40, as will be explained in more detail with reference to Fig. 4 showing a second embodiment of the combination appliance 10. This second embodiment includes a modification over the first embodiment according to Figs. 1 to 3, which mainly concerns the concept for the re-entry of the conveyed air into the atmosphere.

According to Fig. 4, the fan outlet 40 is immediately followed by an outlet duct 58 replacing the air duct 44 and its air duct bending of the first embodiment. Rather than to convey the air through the interior of the kitchen cabinet 16 inside of air duct 44, an immediate rearward air exhaustion shortly after the fan outlet 40 (indicated in Fig. 4 by dotted lines) through the outlet duct 58 and, thereafter, through the exhaust opening 30 on the rear side of the exhaustion device 14 is provided. On its way through the outlet duct 58, the conveyed air passes the odour filter element 60 for the removal of odours from the conveyed air before it re-enters the ambient air on the reverse side of the kitchen cabinet 16. In this respect, the outlet duct 58 includes a receiving space for the odour filter element 60, which receiving space forms a filter seat 62 for an accommodation of the odour filter element 60. For reasons of clarity, only the filter seat is illustrated in Fig. 4 (mainly in dotted lines), but the skilled person will readily understand the way of arrangement of the odour filter element 60 inside of the filter seat 62. Anyway, in order to provide a nearly play-free placing of the odour filter element 60, the filter seat 62 has nearly the same inner shape as the outer contours of the filter element 60, however, with slightly increased dimensions. Naturally, the filter seat 62 comprises open sides in airflow direction enabling a passage of the airflow through nearly the entire filter surface.

With Figs. 5a to 5c the odour filter element 60 is shown isolated from the filter seat 62, e. g. forming a virgin one prepared for being inserted into the extraction device 14, more specifically into the filter seat 62. Fig. 5a is a front view of the odour filter element 60, which is provided when looking from the exhaust opening 30 into the outlet duct 58 with viewing direction straightly onto the extraction fan 36. A cuboid filter frame 64 supports a carbon filter 66, which is formed by a filter fleece that is folded in an undulated way and clamped in the filter frame 64, providing a sufficient filtering effect by this means. A rectangular cover plate 68 (see particularly Fig. 5b) forms a lateral closing of the filter frame 64. Said cover plate 68 also forms a handle for gripping the filter element 60 for its insertion into and removal from the filter seat 62 for servicing processes. In order to improve the gripping security, the handle surface of the cover plate 68 is equipped with ribs 70. Contrary to the open sides in airflow direction, the top side (see Fig. 5c) and the bottom side of the filter frame 64 is fully-surfaced closed.

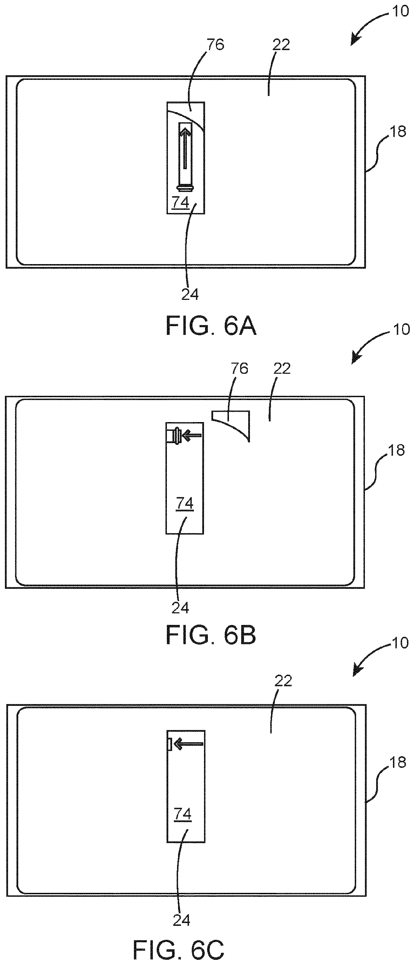

The cover plate 68 of the filter frame 64 surrounded by an O-ring gasket 72 for a radial sealing against a bypass for the air conveyance in axial direction of the filter element 60, as will be more explained now with reference to Figs. 6a to 6c.

As previously mentioned, the odour filter element 60, having a cuboid outer shape, is accommodated in its operating position in the filter seat 62, the inner shape thereof also being of a cuboid shape, but providing a small gap at least in height and width in order to enable a frictionless slide-in insertion of the filter element 60. Access to the filter seat 62 for the filter element 60 is provided through the suction opening 24 after removal of the filter carrier 50 and the filter assembly 48 comprised therein. When removed, the filter carrier 50 releases a receiving space 74 for the filter carrier 50, which is a section of the interior of the casing 28. Said receiving space 74 also functions as an initial receiving space for the odour filter element 60 at the beginning of the insertion process as illustrated in Fig. 6a. The odour filter element 60 is introduced in the receiving space 74 with its longitudinal axis in the insertion movement direction in the initial movement direction, which is similar to or congruent with the cooktop centreline. The longer side of the rectangular cross-section of the filter element 60 is vertically arranged as it is also in the operating position of the filter element 60, i. e. after it has taken on its final position at the end of the insertion process. The orientation of the filter element 60 is such that the cover plate 68, i. e. the handle of the filter element 60, runs after the filter frame 64 during the insertion movement.

As shown in Fig. 4, the operating position of the odour filter element 60 is behind the fan housing 42 from the user's perspective with the longitudinal axis of the odour filter element 60 being in parallel with the longer side of the cooktop 22. Insofar, the final position of the filter element 60 at the end of the insertion movement is orthogonal to the initial orientation at the beginning of the insertion movement. This means that a 90 degrees deflection of the filter element 60 is provided during its insertion. To this end, the filter element 60, in particular its filter frame 64 is constructed with flexible material, which may be an elastic rubber or silicon material. In order to facilitate the deflection process for the user during the insertion movement, the user has put in an insertion aid 76 into the receiving space 74 at the place of redirection prior to the insertion of the filter element 60. Said insertion aid 76 includes a track guide (not shown) at the surface that forms a sliding surface for the filter frame 64 during the insertion movement. By this means, also a lead-in guiding for the filter element 60 into an entrance zone of the filter seat 62 is provided. In order to have a fully guided movement, which prevents the filter element 60 from swerving upwards or downwards, top and bottom guiding surfaces are included as well, therein providing a guiding groove.

Fig. 6b shows the odour filter element 60 already partly inserted in the filter seat 62, which is a stage directly after the deflection phase during the insertion movement. The insertion aid 76 is no longer needed from this moment on, so that the user can remove it from the receiving space 74 (indicated in Fig. 6b). There is only a final insertion actuation left, as indicated by the arrow in Fig. 6b.

Fig. 6c illustrates the odour filter element 60 in its operating position after finalization of the insertion movement. However, in order to fix the filter element 60 in this operation position, a final fixation movement has to be exerted by the user, indicated by the arrow in Fig. 6c. This final fixation movement is a further-pressing in insertion movement against the force of a spring acting opposite to the insertion direction, during which further-pressing a latch means (not shown) engages the filter frame side that is opposite to the cover plate 68, so that an undesired shift of the filter element 60 in the operating position is prevented. The user can then stop exerting force onto the cover plate 68 and the filter element 60 performs a short return movement, where the insertion movement is ultimately finalized.

Fig. 6c also shows that the filter element 60 is entirely implemented in the filter seat 62, so that only an ultimate end section of the cover plate 68 is visible. That also means that the O-ring gasket 72 is also moved inside an entrance zone of the filter seat 62. Said entrance zone is designed in a way that only a small gap is left between the inner surface of the entrance zone and the outer surface of the cover plate 68, which small gap is sealed by the O-ring gasket 72 in an airtight manner. Said sealing by the O-ring gasket 72 is provided not only at the operating position of the filter element 60, but also entirely during the previously described final fixation movement (arrow in Fig. 6c), during which movement the O-ring gasket 72 performs a short sliding movement alongside the inner surface of the entrance zone.

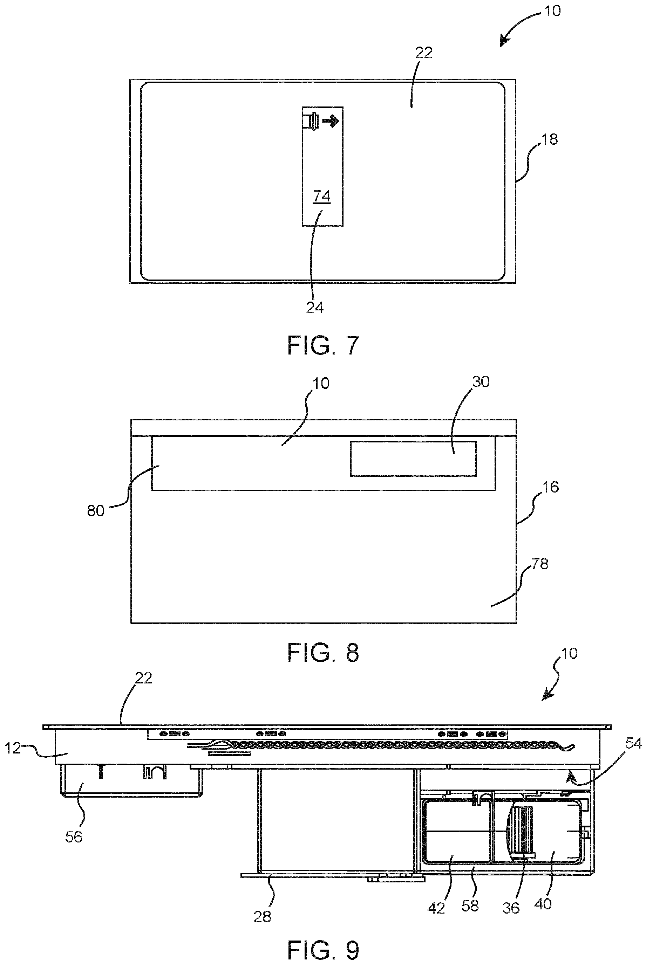

Fig. 7 illustrates an early stage during a removal movement, i. e. when the user e. g. starts a replacement of the odour filter element 60. In order to start the removal movement, the user also presses against the cover plate 68, so that the filter element 60 performs a short movement in insertion direction, which short movement is of equal length as the short return movement at the end of the insertion process. During this short movement the latch means releases the filter frame 64 and the force of the spring pushes the filter element 60 out from the filter seat 62 so far that the user is able to grip the handle, i. e. the cover plate 68 of the filter element 60, and a removal by a pulling action performed by the user is enabled. Said pulling action has to be performed in a way that, at the end thereof, the filter element 60 comes to an arrangement in the receiving space 74 in an arrangement as illustrated in Fig. 6a. Consequently, the user has to exert again a deflection of the filter frame 64, however, due to the pulling action, without the need of using the insertion aid 76. When arrived in the receiving space 74, the filter element 60 can be lifted out thereof and replaced by a new one in the above-describe way according to Figs. 6a to 6c.

The cover plate 68 in combination with the sealing performed by the O-ring gasket functions as an airtight closing wall, which is necessary for a separation of the receiving space 74, arranged upstream of the exhaust fan 36, from the filter seat 62, arranged downstream of the exhaust fan 36. The separation prevents a bypass airflow that would decrease the exhaustion efficiency.

Fig. 8 schematically illustrates the rear side of the kitchen cabinet similar to that one of Fig. 1, but contrary thereto, the exhaust air outlet is directed rearward as has been described with reference to Fig. 4. For this reason, a rear wall 78 of the kitchen cabinet 16 has to be provided with a cutout 80 in order to enable the exhaust air to be blown out of the kitchen cabinet 16 towards a kitchen wall, where the exhaust air spreads over the gap between rear wall 78 and kitchen wall. An ideal structure of the rear side of the extraction device 14 is formed by an entirely flat rear surface, so that the cutout can easily be provided by a kitchen installer by reproducing the rear surface of the extraction device 14 in the rear wall 78 of the kitchen cabinet 16, as illustrated in Fig. 8.

Finally, in comparison to the arrangement of Fig. 3, the concept according to Fig. 9 provides that the extraction fan 36 and the related fan outlet 40 and the outlet duct 54 arranged downstream thereof are moved to a lower position inside of the casing 28, which is favourable for a ventilation inlet for cooling purposes for the power board 54, which ventilation inlet is positioned above the outlet duct 58. With this lower positioning sufficient space can be provided between the outlet duct 58 and the bottom wall of a power board housing, which includes said ventilation inlet, so that sufficient cooling air can pass through the ventilation inlet.

Although illustrative embodiments of the present invention have been described herein with reference to the accompanying drawings, it is to be understood that the present invention is not limited to these precise embodiments, and that various other changes and modifications may be affected therein by one skilled in the art without departing from the scope or spirit of the invention. All such changes and modifications are intended to be included within the scope of the invention as defined by the appended claims.

### List of reference numerals

- 10: combination appliance
- 12: cooking hob
- 14: downdraft extraction device
- 16: kitchen cabinet
- 18: kitchen countertop
- 20a, 20b: cooking regions
- 22: cooktop
- 24: suction opening
- 26: cover grid
- 28: casing
- 30: exhaust opening
- 32: base area
- 34: outlet grille
- 36: extraction fan
- 38: intake opening
- 40: fan outlet
- 42: fan housing
- 44: air duct
- 46^{1 to 5}: arrows indicating air flow
- 48: filter assembly
- 50: filter carrier
- 54: power boards
- 56: control electronics
- 58: outlet duct
- 60: odour filter element
- 62: filter seat
- 64: filter frame
- 66: carbon filter
- 68: cover plate
- 70: rib
- 72: O-ring gasket
- 74: receiving space
- 76: insertion aid
- 78: rear wall
- 80: cutout

## Claims

1. An extraction device (14), in particular an extraction hood, for removing air, specifically cooking vapours, from an area above a cooking hob (12), the extraction device (14) particularly being a downdraft extraction hood and/or particularly forming a combination appliance (10) in combination with the cooking hob (12), the extraction device (14) specifically being arranged below the cooking hob (12), the extraction device (14) comprising
- an air inlet opening (24), which is particularly arranged at or below a cutout or a recess of a worktop (22), specifically of a cooktop or glass plate of the cooking hob (12),
- a fan (36) for aspirating air from the area above the cooking hob (12) through the air inlet opening (24), in particular through the cutout or recess of the worktop (22),
- a filter (60) element, in particular comprising an odour filter, specifically a carbon filter, for removing odours from the aspirated air, and/or a grease particle filter for removing particles from the aspirated air, and/or an electrostatic filter for removing particles, which are based on the electrostatic principle, from the aspirated air,
- an outlet opening (30) for exhausting air, in particular into the ambient air,
**characterized in that**
the filter element (60) is removably arranged in a filter seat (62) downstream of the air inlet opening (24), preferably in the interior of the extraction device (14), and is accessible through the air inlet opening (24), in particular through the cutout or recess of the worktop (22), wherein support means or actuation means are included supporting or facilitating insertion into and/or removal from the filter seat (62), in particular for a manual insertion and/or removal, which support means or actuation means being arranged at and/or acting on the filter element (60), preferably arranged at an end or side of the filter element (60) facing the direction of insertion or removal movement and/or acting on an end or side of the filter element (60), in a direction at least partially opposite to the direction of insertion.

2. The extraction device (14) according to claim 1,
**characterized in that**
the filter element (60) is arranged downstream of the fan (36) and removable through the air inlet opening (24), in particular through the cutout or recess of the worktop (22).

3. The extraction device (14) according to claim 1 or 2,
**characterized in that**
the support means or actuation means comprise a guide mechanism, specifically a sliding-block guide, and/or a spring element configured to at least partially displace the filter element (60) from its operating position that is assumed by the filter element (60) when it is inserted in the filter seat (62), wherein the displacement in particular takes place upon a user's manual operation, the guide mechanism preferably including a cardioid guide mechanism.

4. The extraction device (14) according to anyone of the preceding claims,
**characterized in that**
the filter element (60) is both positionable in and at least partially removable from its operating position within the filter seat (62) by an actuation, specifically by a mechanical action, in the direction of insertion.

5. The extraction device (14) according to anyone of the preceding claims,
**characterized in that**
the support means or actuation means comprise an actuation surface (68), particularly a gripping surface, configured for performing a manual insertion and/or removal movement of the filter element (60).

6. The extraction device (14) according to anyone of the preceding claims,
**characterized by**
a cover (68) separating the filter seat (62) from the air inlet opening (24), in particular from a receiving space (74) directly downstream of the air inlet opening (24).

7. The extraction device (14) according to claim 6,
**characterized in that**
the separation of the filter seat (62) from the air inlet opening (24) is performable in an airtight manner, wherein the cover (68) in particular comprises a sealing means (72), preferably for a radial sealing, the sealing means specifically comprising an O-ring seal.

8. The extraction device (14) according to claim 6 or 7,
**characterized in that**
a transition section is arranged adjacent to and directly upstream of the filter seat (62), which transition section the filter element (60) passes through during its insertion into the filter seat (62), wherein particularly an inner surface of the transition section forms a contact surface for the sealing means.

9. The extraction device (14) according to anyone of the claims 6 to 8,
**characterized in that**
the cover (68) is fixed to and/or integrally formed with the filter element (60), particularly fixed to and/or integrally formed with a frame part (64) of the filter element (60), wherein the cover (68) preferably forms an end portion or end element of the filter element (60) viewed in the direction of insertion movement.

10. The extraction device (14) according to anyone of the preceding claims,
**characterized in that**
the filter element (60) is
- bendable in insertion and/or removal direction for an adaptation to a curved insertion and/or removal path, and/or
- incompressible or only marginally compressible in the direction of its movement.

11. The extraction device (14) according to anyone of the preceding claims,
**characterized by**
a marking or indicator means for pointing out the access to or the position of the filter element (60) to the user, which marking or indicator means
- is visible or noticeable through the air inlet opening (24),
- particularly provides a visual and/or haptic guidance to the user,
- is preferably located on a bottom cover or an intermediate bottom wall of the extraction device (14).

12. The extraction device (14) according to anyone of the preceding claims,
**characterized by**
an insertion aid (76) for the facilitated insertion of the filter element (60) into the filter seat (62), the insertion aid (76) particularly comprising
- a rail or track guide means, and/or
- at least one guiding surface, preferably at least two guiding surfaces, more preferably three guiding surfaces.

13. The extraction device (14) according to claim 12,
**characterized in that**
the insertion aid (76)
- is an integral element of the extraction device (14), or
- is insertable into the extraction device (14), in particular through the air inlet opening (24), prior to the filter element insertion process, and is removable from the extraction device (14) after finalization of the filter element insertion process.

14. The extraction device (14) according to anyone of the preceding claims,
**characterized in that**
the filter element (60) is insertable into and/or removable from the filter seat (62) in an at least approximately horizontal direction and the horizontal extension of the filter element (60) in its direction of movement is equal to or greater than its vertical extension, the vertical cross-section of the filter element (60) preferably being rectangular or oval.

15. The extraction device (14) according to anyone of the preceding claims,
**characterized by**
purification means for a cleaning of the aspirated air, the purification means being formed by
- silver ions included in the filter element (60), and/or
- lighting means emitting ultraviolet rays and being included in the extraction device (14), which lighting means irradiate the filter means at least during the operating periods or at least one time range of an operating period of the extraction device (14).
